# EUROPEAN PATENT SPECIFICATION

(11) **EP 3 133 158 B1**
(45) Date of publication and mention of the grant of the patent: **29.08.2018**
(21) Application number: 15758364.2
(22) Date of filing: 03.03.2015
(51) Int. Cl.: C12N 15/00, C12N 1/15, C12N 1/19, C12N 1/21, C12N 5/10, C12N 9/88, C12N 15/09, C12N 15/60

(54) **NOVEL (R)-HYDROXYNITRILE LYASE**
NEUARTIGE (R)-HYDROXYNITRIL-LYASE
NOUVELLE (R)-HYDROXYNITRILE LYASE

(30) Priority: 04.03.2014 JP 2014042181
(43) Date of publication of application: 22.02.2017
(73) Proprietor: Public University Corporation Toyama Prefectural University, Toyama 9390398 (JP)
(72) Inventor: ASANO, Yasuhisa, Imizu-shi Toyama 939-0398 (JP); MOHAMMAD DADASHIPOUR LAKMEHSARI, Imizu-shi Toyama 939-0398 (JP); ISHIDA, Yuko, Imizu-shi Toyama 939-0398 (JP)
(74) Representative: Müller-Boré & Partner Patentanwälte PartG mbB
(86) International application number: PCT/JP2015/056179
(87) International publication number: WO 2015/133462

(56) References cited:
- US-A1- 2005 064 552
- BREITHAUPT H ET AL: "CLONING AND EXPRESSION OF (R)-HYDROXYNITRILE LYASE FROM LINUM USITATISSIMUM (FLAX)", JOURNAL OF MOLECULAR CATALYSIS. B, ENZYMATIC, ELSEVIER, AMSTERDAM, NL, vol. 6, no. 3, 11 March 1999 (1999-03-11), pages 315-332, XP000995739, ISSN: 1381-1177, DOI: 10.1016/S1381-1177(98)00109-X
- KUWAHARA YASUMASA ET AL: "Release of Hydrogen Cyanide via a Post-secretion Schotten-Baumann Reaction in Defensive Fluids of Polydesmoid Millipedes", JOURNAL OF CHEMICAL ECOLOGY, vol. 37, no. 3, March 2011 (2011-03), pages 232-238, XP002770797,
- DADASHIPOUR MOHAMMAD ET AL: "Discovery and molecular and biocatalytic properties of hydroxynitrile lyase from an invasive millipede, Chamberlinius hualienensis", PROCEEDINGS OF THE NATIONAL ACADEMY OF SCIENCES OF THE UNITED STATES OF AMERICA, vol. 112, no. 34, August 2015 (2015-08), pages 10605-10610, XP002770798, ISSN: 0027-8424
- M. DADASHIPOUR ET AL: "hydroxynitrile lyases: Insights into biochemistry, discovery, and engineetingv", AMERICAN CHEMICAL SOCIETY CATALYSIS, vol. 1, 2011, pages 1121-1149, XP002770799,
- DUFFEY S. S. ET AL.: 'On the biochemical basis of HCN production in the millipede Harpaphe haydeniana (Xystodesmidae: Polydesmida' CANADIAN JOURNAL OF ZOOLOGY vol. 56, no. 1, 1978, pages 7 - 16, XP008184247
- DADASHIPOUR L. M. ET AL.: 'A new hydroxynitrile lyase: purification, characterization and recombinant expression' JAPAN SOCIETY FOR BIOSCIENCE, BIOTECHNOLOGY, AND AGROCHEMISTRY 2014 NENDO TAIKAI KOEN YOSHISHU 05 March 2014, page 3D03A08, XP008184363
- DADASHIPOUR L. M. ET AL.: 'Hydroxynitrile lyase from a millipede as a valuable tool for white biotechnology: purification, characterization and recombinant expression' JAPANESE SOCIETY OF ENZYME ENGINEERING DAI 72 KAI KOENKAI YOSHISHU December 2014, pages 41 - 42, XP008184839

## Description

### TECHNICAL FIELD

The present invention relates to a (R)-hydroxynitrile lyase, a gene which encodes the hydroxynitrile lyase, and a production method of the hydroxynitrile lyase. The stability of the (R)-hydroxynitrile lyase is superior to that of a hydroxynitrile lyase derived from a plant, and heterologous expression is possible by the gene.

### BACKGROUND ART

A hydroxynitrile lyase is an enzyme which catalyzes a reaction to convert a carbonyl compound to a cyanohydrin, i.e. α-hydroxynitrile, in the presence of a cyanide donor. A cyanohydrin is important as an intermediate in the field of pharmaceuticals, chemicals or the like, since a cyanohydrin can be converted to various compounds such as α-hydroxy acid, α-hydroxy ketone and β-amino alcohol. It has been therefore desired to develop a method for producing a large amount of a hydroxynitrile lyase.

A hydroxynitrile lyase is classified into (S)-selective group and (R)-selective group. A (R)-hydroxynitrile lyase catalyzes a reaction to generate (R)-cyanohydrin from a ketone compound or an aldehyde compound and a cyanide compound in acidic condition. As a representative example of the reaction, a reaction to generate (R)-mandelonitrile from benzaldehyde and hydrocyanic acid as a cyanide compound is exemplified. In addition, a (R)-hydroxynitrile lyase is very useful in various fields as a (R)-hydroxynitrile lyase is also used as a biocatalyst which can produce an optically active substance from an inexpensive substrate. An optically active substance is highly useful as a pharmaceutical intermediate and a chemical intermediate.

In order to utilize a hydroxynitrile lyase for industrial production of an optically active cyanohydrin, it has been desired to develop a method for producing a large amount of a hydroxynitrile lyase which has excellent activity per fungus body or peptide and which has high stereospecificity.

It has been known that a hydroxynitrile lyase is mainly contained in a plant which produces a cyanogen glycoside. For example, a (R)-hydroxynitrile lyase derived from a plant of the family Rosaceae, such as almond (Prunus amygdalus), is known. In addition, (S)-hydroxynitrile lyases derived from a plant of the family Gramineae, such as sorghum (Sorghum bicolor), and the family Euphorbiaceae, such as cassava (Manihot esculenta), para rubber tree (Hevea brasiliensis) and Baliospermum are known. However, only a small amount of a hydroxynitrile lyase can be extracted from the above-described plants. However S.S. Duffet ET AL (Canadian Journal of Zoology, vol.56, 1978, 7-16) detects an hydroxynitrile lyase in the millipede Harpaphe haydeniana.

In order to produce a large amount of a hydroxynitrile lyase, an attempt to produce a hydroxynitrile lyase by genetic engineering has been made (Patent Documents 1 to 9). And also H. Breihaupt ET AL (Journal of Molecular Catalysis B: Enzymatic 6, 1999, 315-332) disclosing the cloning and expression of (R)-hydroxynitrile lyase from Linum usitatissimum. the result such as expression amount and biochemical activity obtained by the research using a homologous protein cannot be directly applied to a heterologous protein produced using a transformant in some cases. In other words, when a heterologous protein is produced using a transformant, it is not easy to preliminarily predict a behavior of the transformant, expression amount, biochemical activity of a target protein, or the like.

### PRIOR ART DOCUMENT

### PATENT DOCUMENT

Patent Document 1: JP H11-508775 T
Patent Document 2: JP 2000-189159 A
Patent Document 3: JP 2000-189160 A
Patent Document 4: JP 2000-245486 A
Patent Document 5: JP 2002-330791 A (US2005064552)
Patent Document 6: WO 01/48178
Patent Document 7: JP 2004-194550 A
Patent Document 8: JP 2004-194551 A
Patent Document 9: JP 2000-125886 A

### DISCLOSURE OF THE INVENTION

### PROBLEMS TO BE SOLVED BY THE INVENTION

As described above, since a hydroxynitrile lyase may be industrially very important enzyme, it is required to stably provide a hydroxynitrile lyase for industrial use. However, when a hydroxynitrile lyase is tried to be purified from a plant having a cyanogen glycoside, the obtained amount is very small. In addition, heterologous expression of a hydroxynitrile lyase is difficult. Furthermore, when an optically active compound is produced using a hydroxynitrile lyase, it is necessary to bring about severe conditions to the hydroxynitrile lyase; therefore, highly stable hydroxynitrile lyase is desired.

Under the above-described circumstances, the objective of the present invention is to provide a (R)-hydroxynitrile lyase which is more stable than a hydroxynitrile lyase derived from a plant, a gene which encodes the hydroxynitrile lyase and by which heterologous expression is possible, and a method for producing the hydroxynitrile lyase.

### MEANS FOR SOLVING THE PROBLEMS

The inventors of the present invention made extensive studies to solve the above problems. As a result, the inventors completed the present invention by finding that the (R)-hydroxynitrile lyase produced by Chamberlinius hualienensis, which periodically swarms over in a particular area, is very stable.
[1] A (R)-hydroxynitrile lyase having any one of the following amino acid sequences (1) to (3):
   (1) an amino acid sequence of SEQ ID NO: 3;
   (2) an amino acid sequence specified in the (1) with deletion, substitution and/or addition of 1 or more and 30 or less amino acid residues, wherein a hydroxynitrile lyase activity of a (R)-hydroxynitrile lyase having the amino acid sequence (2) is the same as or superior to that of natural (R)-hydroxynitrile lyase having the amino acid sequence of SEQ ID NO: 3;
   (3) an amino acid sequence having at least 70% sequence homology to the amino acid sequence specified in the (1), wherein a hydroxynitrile lyase activity of a (R)-hydroxynitrile lyase having the amino acid sequence (3) is the same as or superior to that of natural (R)-hydroxynitrile lyase having the amino acid sequence of SEQ ID NO: 3.
[2] The (R)-hydroxynitrile lyase according to the above [1], being a dimer of a subunit having any one of the amino acid sequences (1) to (3).
[3] The (R)-hydroxynitrile lyase according to the above [1] or [2], derived from genus Chamberlinius.
[4] The (R)-hydroxynitrile lyase according to the above [3], derived from Chamberlinius hualienensis.
[5] A (R)-hydroxynitrile lyase gene having any one of the following base sequences (4) to (6):
   (4) a base sequence of SEQ ID NO: 2;
   (5) a base encoding the amino acid sequence (2) according to claim 1;
   (6) a base sequence encoding the amino acid sequence (3) according to claim 1.
[6] The (R)-hydroxynitrile lyase gene according to the above [5], having a base sequence of SEQ ID NO: 1.
[7] A vector, comprising the (R)-hydroxynitrile lyase gene according to the above [5] or [6].
[8] A transformant, transformed by the vector according to the above [7].
[9] A method for producing a (R)-hydroxynitrile lyase, comprising the steps of:
   cultivating the transformant according to the above [8] to obtain a culture, and
   purifying the (R)-hydroxynitrile lyase from the culture.

### EFFECT OF THE INVENTION

The (R)-hydroxynitrile lyase according to the present invention is contained in an individual of Chamberlinius hualienensis, but the amount thereof is small. However, since Chamberlinius hualienensis periodically swarms over in a particular area, Chamberlinius hualienensis can be easily obtained in a relatively large amount as a raw material for enzyme purification. In other words, it is difficult to purify the (R)-hydroxynitrile lyase according to the present invention; however, such a difficulty can be overcome by the large amount of Chamberlinius hualienensis as a raw material. In addition, for example, the (R)-hydroxynitrile lyase according to the present invention can be used for producing a cyanohydrin, which is important as a synthetic intermediate, since the stability of the (R)-hydroxynitrile lyase is excellent. The (R)-hydroxynitrile lyase according to the present invention is therefore very useful industrially.

### BRIEF DESCRIPTION OF THE DRAWINGS

Figure 1 are photographs of Chamberlinius hualienensis, from which the enzyme of the present invention can be isolated. Figure 1(A) is a photograph of a captured relatively big Chamberlinius hualienensis, and Figure 1(B) is a photograph of a relatively small Chamberlinius hualienensis.
Figure 2 are photographs of a gel to demonstrate the result of the analysis of the (R)-hydroxynitrile lyase purified from Chamberlinius hualienensis by SDS-PAGE. Figure 1(A) is a photograph of a gel to measure the molecular mass using molecular-weight markers, and Figure 1(B) is a photograph of a gel stained to determine whether sugar chain was present or not.
Figure 3(A) to (C) are respectively the measurement results of ultraviolet·visible·near-infrared spectrum, infrared spectrum and circular dichroism spectrum of the (R)-hydroxynitrile lyase purified from Chamberlinius hualienensis.
Figure 4 represents cDNA, deduced amino acid sequence and an annealing site of primers of the (R)-hydroxynitrile lyase purified from Chamberlinius hualienensis.
Figure 5(A) and (B) are graphs to demonstrate the relations of the reaction pH with the specific activity or the remaining activity of the (R)-hydroxynitrile lyase purified from Chamberlinius hualienensis.
Figure 6(A) and (B) are graphs to demonstrate the relations of the reaction temperature with the specific activity or the remaining activity of the (R)-hydroxynitrile lyase purified from Chamberlinius hualienensis.
Figure 7(A) and (B) are graphs to demonstrate the relations of the reaction pH or the reaction temperature with the remaining activity of the (R)-hydroxynitrile lyase purified from Chamberlinius hualienensis and recombinant (R)-hydroxynitrile lyase.

### MODE FOR CARRYING OUT THE INVENTION

Hereinafter, the (R)-hydroxynitrile lyase according to the present invention is described.

### <(R)-hydroxynitrile lyase>

The (R)-hydroxynitrile lyase according to the present invention has any one of the above-described amino acid sequences (1) to (3).

The "amino acid sequence of SEQ ID NO: 3" of the above-described (R)-hydroxynitrile lyase is an amino acid sequence of the natural hydroxynitrile lyase derived from Chamberlinius hualienensis. Chamberlinius hualienensis periodically swarms over to stop a train particularly in Kagoshima Prefecture. In addition, Chamberlinius hualienensis has become a problem, since Chamberlinius hualienensis causes health hazard by releasing gas containing hydrogen cyanide when being exterminated. The present inventors noted that Chamberlinius hualienensis produces hydrogen cyanide and considered that Chamberlinius hualienensis may have a hydroxynitrile lyase. The present inventors performed experiment on the basis of the idea; as a result, succeeded in the isolation/purification of the (R)-hydroxynitrile lyase of the present invention. In addition, the present invention is highly regarded as a disposal method of Chamberlinius hualienensis, since the (R)-hydroxynitrile lyase of the present invention can be isolated and purified from Chamberlinius hualienensis, which swarms over and of which extermination method has not been established.

In addition, Nedyopus tambanus tambanus, Parafontaria tonominea, Epanerchodus sp., Epanerchodus fulvus Haga, Parafontaria laminate armigera, Oxidus gracilis, Cryptocorypha sp., Epanerchodus japonicas Carl, Riukiaria semicircularis semicircularis and Nedyopus patrioticus patrioticus, which are classified into the order Polydesmida as Chamberlinius hualienensis, also produce hydrogen cyanide and mandelonitrile as a defensive substance (Kuwahara et al., J. Chem. Ecol., 37, pp.232-238 (2011)). Further, it was confirmed by the preliminary experiment by the present inventors that the homogenate of Parafontaria tonominea and Riukiaria semicircularis semicircularis respectively has (R)-hydroxynitrile lyase activity of 4.23 U/mg and 6.35 U/mg to synthesize mandelonitrile from benzaldehyde. It is therefore considered that the above-described millipedes produce the (R)-hydroxynitrile lyase of the present invention and the (R)-hydroxynitrile lyase of the present invention can be purified from the above-described millipedes.

In the present invention, the term "hydroxynitrile lyase activity" means any one of synthetic activity and degradative activity. In the present invention, "synthetic activity" means an activity to catalyze the reaction to generate a cyanohydrin from a cyanogen compound and a ketone compound or an aldehyde compound, and "degradative activity" means an activity to catalyze the reverse reaction. For example, the synthetic activity according to the present invention can be calculated by measuring the generation amount of (R)-mandelonitrile from benzaldehyde. For example, the generation amount of (R)-mandelonitrile can be quantitated by HPLC. The degradative activity can be calculated by measuring the generation amount of benzaldehyde from mandelonitrile as a substrate. For example, the generation amount of benzaldehyde can be quantitated by measuring the increment of absorbance value at a wavelength of 280 nm when hydroxynitrile lyase and racemic mandelonitrile are added into a sodium citrate buffer.

The (R)-hydroxynitrile lyase according to the present invention has high stability as the activity thereof is maintained particularly in broad pH range and temperature range.

In the present invention, the phrase "an enzyme has a specific amino acid sequence" means that the amino acid sequence of the enzyme contains the specific amino acid sequence and the function of the enzyme is maintained. The sequence of an enzyme other than the specific amino acid sequence is exemplified by a bridging structure such as disulfide bond in addition to histidine tag and a linker sequence for immobilization. In addition, an enzyme may have a sugar chain as long as the enzyme has the specific amino acid sequence.

In the above-described amino acid sequence (2) according to the present invention, the range of "1 or more" in the phrase "an amino acid sequence with deletion, substitution and/or addition of 1 or more amino acid residues" is not particularly restricted as long as the hydroxynitrile lyase activity of the (R)-hydroxynitrile lyase having the above-described deletion, substitution and/or addition is the same as or superior to the activity of natural hydroxynitrile lyase such as the natural (R)-hydroxynitrile lyase having the above-described amino acid sequence (1). The range of the above-described "1 or more" may be 1 or more and 30 or less, preferably 1 or more and 20 or less, more preferably 1 or more and 10 or less, more preferably 1 or more and 7 or less, even more preferably 1 or more and 5 or less, and particularly preferably 1 or more and 3 or less, 1 or more and 2 or less, or 1.

In the above-described amino acid sequence (3) according to the present invention, the range of "sequence homology" in the phrase "an amino acid sequence having at least 70% sequence homology to the amino acid sequence specified in the (1)" is not particularly restricted as long as the hydroxynitrile lyase activity of the (R)-hydroxynitrile lyase having the above-described sequence homology the same as or superior to the activity of the natural (R)-hydroxynitrile lyase having the amino acid sequence of SEQ ID NO: 3. The above-described sequence homology of an amino acid sequence is not restricted as long as the value is 70% or more, or 80% or more, more preferably 90% or more, 92% or more, 94% or more, or 95% or more, even more preferably 96% or more, 98% or more, 99% or more, or 99.5% or more, and particularly preferably 99.8% or more. In the present invention, the term "sequence homology of sequence" means the degree of amino acid homology between two or more amino acid sequences. When a homology of certain two amino acid sequences is higher, the homology and analogy of the sequences are high. It can be analyzed whether or not two amino acid sequences share a specific homology by directly comparing the sequences, specifically by using commercially available sequencing analysis software.

Regardless of the homology search using Blastp by the present inventors, conventionally-known protein having homology to the (R)-hydroxynitrile lyase of the present invention has not been searched. Specifically, only five proteins were searched by Blastp. Among the proteins, it was confirmed that BAD30832 hypothetical protein [Oryza sativa Japonica Group] has the highest homology; however, the homology value is only 44%. In addition, the homology value was measured by comparing with partial sequences having 29 amino acid residues. If the amino acid sequences of whole proteins are compared, the homology value is supposed to be much smaller. As the above search result, the (R)-hydroxynitrile lyase of the present invention does not have homology to conventionally-known proteins and is considered to be novel.

In the definition of the above-described amino acid sequences (2) and (3), the phrase "a hydroxynitrile lyase activity of a (R)-hydroxynitrile lyase is the same as or superior to that of natural (R)-hydroxynitrile lyase having the amino acid sequence of SEQ ID NO: 3" means that the hydroxynitrile lyase activity of an object protein is relatively the same or superior to the activity of the natural (R)-hydroxynitrile lyase having the amino acid sequence of SEQ ID NO: 3. Specifically, the above-described synthetic reaction or degradative reaction is performed using two or more enzymes to be compared each other in the same condition, and the amount of generated compound is compared. If the activity of the enzyme having the above-described amino acid sequence (2) or (3) is higher than that of the above-described natural enzyme, the above-described requirement is met.

### <Gene and vector>

The vector according to the present invention encodes the above-described (R)-hydroxynitrile lyase.

The above-described gene (4) encodes the natural (R)-hydroxynitrile lyase which is isolated and purified from Chamberlinius hualienensis. The above-described gene (4) may be therefore obtained by PCR in which cDNA prepared from Chamberlinius hualienensis is used as a template.

The base sequences (5) and (6) of the gene according to the present invention can be designed as a base sequence which encodes the amino acid sequence of the above-described (R)-hydroxynitrile lyase (2) or (3).

For example, the gene of the present invention can be obtained from cDNA library using the (R)-hydroxynitrile lyase gene DNA having the base sequence of SEQ ID NO:1 or SEQ ID NO:2, a complementary sequence thereof or a fragment thereof as a probe by already-known hybridization method such as colony hybridization technique and Southern blot technique. Alternatively, the DNA having the base sequence of SEQ ID NO:1 or SEQ ID NO:2 may be synthesized by a conventional gene synthesis method.

In the present invention, the "stringent condition" means a condition for washing after hybridization, for example, a salt concentration of 300 mM or more and 2000 mM or less, and a temperature of 40°C or higher and 75°C or lower, preferably a salt concentration of 600 mM or more and 900 mM or less, and a temperature of 65°C. The stringent condition is exemplified by a condition of 2×SSC at 50°C. A person skilled in the art can set the condition to obtain the nucleic acid which encodes the (R)-hydroxynitrile lyase of the present invention, such as a probe concentration, a length of a probe and reaction time in addition to the above-described salt concentration and temperature of a buffer.

With respect to the detailed procedure of the hybridization method, "Molecular Cloning, A Laboratory Manual 2nd ed." Cold Spring Harbor Laboratory Press (1989) or the like can be referred. The nucleic acid for hybridization is exemplified by a nucleic acid and a fragment thereof which have the base sequence having a homology of at least 40%, preferably 60% or more, more preferably 90% or more to the base sequence of SEQ ID NO:1 or SEQ ID NO:2.

In the present invention, the method for producing the (R)-hydroxynitrile lyase gene is not particularly restricted, and the gene may be produced by a conventionally-known method. As such a conventionally-known method, exemplified are a method of introducing a site-specific mutation to natural (R)-hydroxynitrile lyase gene using a commercially available kit, a method of selectively cleaving the gene DNA and then removing and adding the selected oligonucleotide to be ligated, or the like.

The above-described site-specific mutagenesis method is described in "Molecular Cloning, A Laboratory Manual 2nd ed." Cold Spring Harbor Press (1989); "Current Protocols in Molecular Biology" John Wiley & Sons (1987-1997); Kunkel, Proc. Natl. Acad. Sci. USA, 82, pp.488-92 (1985); Kramer and Fritz, Method. Enzymol., 154, pp.350-67 (1987); Kunkel, Method. Enzymol., 85, pp.2763-6 (1988) or the like. Recently, a site-specific mutagenesis method can be performed by using a mutation introducing kit based on Kunkel method and Gapped duplex method, such as QuikChange™ Site-Directed Mutagenesis Kit (manufactured by Stratagene), GeneTailor™ Site-Directed Mutagenesis System (manufactured by Invitrogen), TaKaRa Site-Directed Mutagenesis System such as Mutan-K and Mutan-Super Express Km (manufactured by Takara Bio Inc.).

When the target site to be introduced by mutation is close to the cleavage site which is easily cleaved by a restriction enzyme and at which ligation is easily conducted in a target gene sequence, a gene DNA fragment into which a target mutation is introduced can be obtained without difficulty by PCR using a primer which is a synthesized oligo DNA and into which a target mutation is introduced. In addition, the gene DNA fragment can be obtained as a synthesized gene by assembly PCR in which synthesized oligo DNAs are combined to be used.

In addition, the desired mutant-type (R)-hydroxynitrile lyase gene can be obtained from natural (R)-hydroxynitrile lyase gene by a method for introducing mutation in a random manner. Such a method is exemplified by a method of contacting and reacting a reagent such as hydroxylamine and nitrite for mutation, a method of mutagenesis by ultraviolet irradiation, a method of random mutagenesis by polymerase chain reaction (i.e. PCR), or the like.

In order to express the (R)-hydroxynitrile lyase gene according to the present invention obtained by the above-described method in a host, an expression cassette is prepared by inserting a transcription promoter upstream of the gene and a terminator downstream of the gene, and the cassette is inserted into an expression vector. Alternatively, when there are a transcription promoter and a terminator in an expression vector to be inserted by the improved hydroxynitrile lyase gene, the mutated gene may be inserted between the promoter and terminator in the vector without preparing an expression cassette. In order to insert the improved hydroxynitrile lyase gene into a vector, a method of using a restriction enzyme, topoisomerase or the like may be employed. In addition, an appropriate linker may be added, if it is necessary for the insertion. In the present invention, the above-described integration procedure may be performed while the (R)-hydroxynitrile lyase gene is prepared. Specifically, PCR is carried out by using a primer having a base sequence substituted by a base sequence which encodes other amino acid and using a recombinant vector in which natural hydroxynitrile lyase gene is cloned as a template, and the obtained amplified product is incorporated with a vector.

The kind of a promoter is not particularly restricted as long as the promoter enable an appropriate expression in a host. A promoter is exemplified by trp promoter of a tryptophan operon derived from Escherichia coli, lac promoter derived from a lactose operon, PL promoter and PR promoter derived from lambda phage, gluconate synthetase promoter (gnt) derived from Bacillus subtilis, alkaline protease promoter (apr), neutral protease promoter (npr) and α-amylase promoter (amy). In addition, improved sequence or designed sequence, such as tac promoter and trc promoter, can be also used.

A terminator is not necessarily essential, and the kind thereof is not particularly restricted. A terminator is exemplified by a ρ factor-independent terminator such as lipoprotein terminator, trp operon terminator and rrnB terminator.

As an important base sequence for translation to an amino acid, ribosome-binding sequence such as SD sequence and Kozak sequence is known. Such a sequence may be inserted upstream of the mutated gene. By PCR method, SD sequence may be added when a prokaryotic organism is used as a host, and Kozak sequence may be added when a eukaryotic cell is used as a host. SD sequence is exemplified by a sequence derived from Escherichia coli and Bacillus subtilis, but is not particularly restricted as long as the sequence works in a desired host such as Escherichia coli and Bacillus subtilis. For example, a consensus sequence having continuous four or more sequences which are complementary to 3' end region of 16S ribosomal RNA may be prepared by DNA synthesis to be used.

In general, a vector contains a selection marker, which is a factor to select a target transformant. A selection marker is exemplified by drug resistance gene, auxotrophic complementary gene and gene for imparting assimilating property, and may be selected depending on the purpose or a host. For example, drug resistance gene used as a selection marker for Escherichia coli is exemplified by ampicillin-resistant gene, kanamycin gene, dihydrofolate reductase gene and neomycin-resistant gene.

The vector used in the present invention is not particularly restricted as long as the vector can maintain the above-described mutated gene. The vector which conforms to the used host may be used. The vector is exemplified by plasmid DNA, bacteriophage DNA, retrotransposon DNA and artificial chromosome DNA. For example, when Escherichia coli is used as a host, a vector which has a region capable of autonomous replicating in Escherichia coli, such as pTrc99A (Centraalbureau voor Schimmelcultures (CBS), the Netherlands; http://www.cbs.knaw.nl/), pUC19 (Takara Bio Inc., Japan), pKK233-2 (Centraalbureau voor Schimmelcultures (CBS), the Netherlands; http://www.cbs.knaw.nl/), pET-12 (Novagen, Germany) and pET-26b (Novagen, Germany), may be used. In addition, if necessary, the vector may be altered to be used. Furthermore, a vector having high expression efficiency may be used. Such a vector is exemplified by expression vector pTrc99A and pKK233-2, which have trc promoter and lac operator.

The recombinant vector which contains the above-described improved hydroxynitrile lyase gene is included in the range of the present invention.

### <Transformant>

A transformant can be produced by transducing the recombinant vector of the present invention into a host to be transformed. Such a transformant is also included in the range of the present invention.

A host used in the present invention is not particularly restricted as long as the target (R)-hydroxynitrile lyase can be produced by introducing the above-described recombinant vector into the host. The host is exemplified by a bacterium such as Escherichia coli and Bacillus subtilis; a fungus belonging to genus Pichia such as Pichia pastoris, genus Saccharomyces, genus Aspergillus or the like; an animal cell such as HEK293; an insect cell such as Sf9 cell, Sf21 cell and High Five (BTI-TN-5B1-4); and a plant cell.

### <Production method of (R)-hydroxynitrile lyase>

The (R)-hydroxynitrile lyase of the present invention can be produced by cultivating the above-described transformant to obtain a culture and purifying the (R)-hydroxynitrile lyase from the culture.

In the present invention, the term "culture" means any one of culture supernatant, cultured cell, cultured bacteria/fungus body and homogenate of cell or bacteria/fungus body. The culture obtained by cultivating the transformant of the present invention is included in the range of the present invention.

It is performed by a general method for cultivating a host to cultivate the transformant of the present invention. The target (R)-hydroxynitrile lyase is accumulated in the above-described culture.

The culture medium to cultivate the transformant of the present invention contains carbon source, nitrogen source, an inorganic salt or the like which are assimilated by a host. The culture medium may be any one of natural medium and synthetic medium as long as the transformant can be efficiently cultivated in the culture medium. The carbon source is exemplified by a carbohydrate such as glucose, galactose, fructose, sucrose, raffinose and starch; an organic acid such as acetic acid and proprionic acid; and an alcohol such as ethanol and propanol. The nitrogen source is exemplified by an ammonium salt of an inorganic acid and an organic acid, and other nitrogen-containing compound, such as ammonia, ammonium chloride, ammonium sulfate, ammonium acetate and ammonium phosphate. In addition, peptone, yeast extract, meat extract, corn steep liquor, various amino acids, or the like may be used. The inorganic salt is exemplified by potassium dihydrogenphosphate, dipotassium hydrogenphosphate, magnesium phosphate, magnesium sulfate, sodium chloride, ferrous sulfate, manganese sulfate, zinc sulfate, copper sulfate and calcium carbonate. If necessary, a defoaming agent may be added to inhibit foaming during the cultivation. In addition, vitamin and the like may be appropriately added, if needed. During the cultivation, an antibiotic agent such as ampicillin and tetracycline may be added into the culture medium as appropriate.

The cultivation may be performed while selective pressure is applied in order to prevent the vector and the target gene from being detachably transferred. Specifically, when a selection marker is a drug resistance gene, the corresponding drug may be added to the culture medium; and when a selection marker is an auxotrophic complementary gene, the corresponding nutritional factor may be removed from the culture medium. In addition, when a selection marker is a gene for imparting assimilating property, the corresponding factor to be assimilated may be added as only one factor as needed. For example, when Escherichia coli which is transformed by a vector containing ampicillin-resistant gene is cultivated, ampicillin may be added into the culture medium as necessary.

When a transformant which is transformed by an expression vector containing an inducible promoter as a promoter is cultivated, an inducer may be added into the culture medium as appropriate. For example, when a transformant which is transformed by an expression vector containing a promoter inducible by isopropyl-β-D-thiogalactoside (IPTG) is cultivated, IPTG or the like may be added into the culture medium. In addition, when a transformant which is transformed by an expression vector containing a promoter inducible by indoleacetic acid (IAA) is cultivated, IAA or the like may-be added into the culture medium.

The condition for the cultivation of the transformant is not particularly restricted as long as it is not inhibited to produce the target (R)-hydroxynitrile lyase and to grow the host. In general, the cultivation temperature may be 10°C or higher and 45°C or lower, preferably 10°C or higher and 40°C or lower, more preferably 15°C or higher and 40°C or lower, and even more preferably 20°C or higher and 37°C or lower. The temperature may be changed during the cultivation as necessary. The cultivation time may be about 5 hours or more and about 120 hours or less, preferably 5 hours or more and 100 hours or less, more preferably 10 hours or more and 100 hours or less, and even more preferably 15 hours or more and 80 hours or less. The pH may be adjusted by using inorganic acid, organic acid, an alkaline solution or the like, and is adjusted to 6 or more and 9 or less when Escherichia coli is cultivated. A cultivation method is exemplified by solid cultivation, static cultivation, shaking cultivation and aeration-agitation cultivation.

The initial pH of the culture medium for the cultivation may be appropriately adjusted to 7 or more and 9 or less. The cultivation may be performed at 5°C or higher and 40°C or lower, preferably 10°C or higher and 37°C or lower, for 5 hours or more and 100 hours or less. The cultivation is preferably performed by aeration-agitation deep cultivation, shaking cultivation, static cultivation or fed-batch cultivation.

The (R)-hydroxynitrile lyase of the present invention is purified from the above-described culture, and the purification degree is not particularly restricted. For example, the above-described culture is homogenized, an insoluble component is removed from the homogenate by filtration, centrifugation or the like to obtain a solution, and the solution may be directly used. The (R)-hydroxynitrile lyase may be further purified to obtain a crude enzyme solution or an enzyme solution.

### <Enzyme reaction>

The (R)-hydroxynitrile lyase catalyzes a reaction to transform an aldehyde and a ketone into a cyanohydrin in the presence of a cyanide donor, and the reverse reaction thereof, i.e. decomposition reaction of a cyanohydrin.

In the present invention, for example, the activity of the hydroxynitrile lyase is measured by using benzaldehyde as a substrate in the presence of potassium cyanide and measuring production amount of mandelonitrile with HPLC. Alternatively, a method in which racemic mandelonitrile is used as a substrate and the decomposition degree is detected by measuring absorbance of absorption wavelength of 280 nm using an absorption spectrometer is exemplified. It is preferred to use a sodium citrate buffer as a solvent, since the reaction mixture should be more acidic than neutral for the stability of cyanohydrin. The synthetic reaction is preferably performed at pH of about 4.0 or more and about 4.2 or less, and the decomposition reaction is preferably performed at pH of about 5 or more and about 5.5 or less.

The present application claims the benefit of the priority date of Japanese patent application No. 2014-42181 filed on March 4, 2014.

### EXAMPLES

Hereinafter, the present invention is described in more detail with Examples. However, the present invention is not restricted to the following Examples in any way, and it is possible to work the present invention according to the Examples with an additional appropriate change within the range of the above descriptions and the following descriptions. Such a changed embodiment is also included in the technical scope of the present invention.

### Example 1: Production of natural (R)-hydroxynitrile lyase

### (1) Purification of (R)-hydroxynitrile lyase

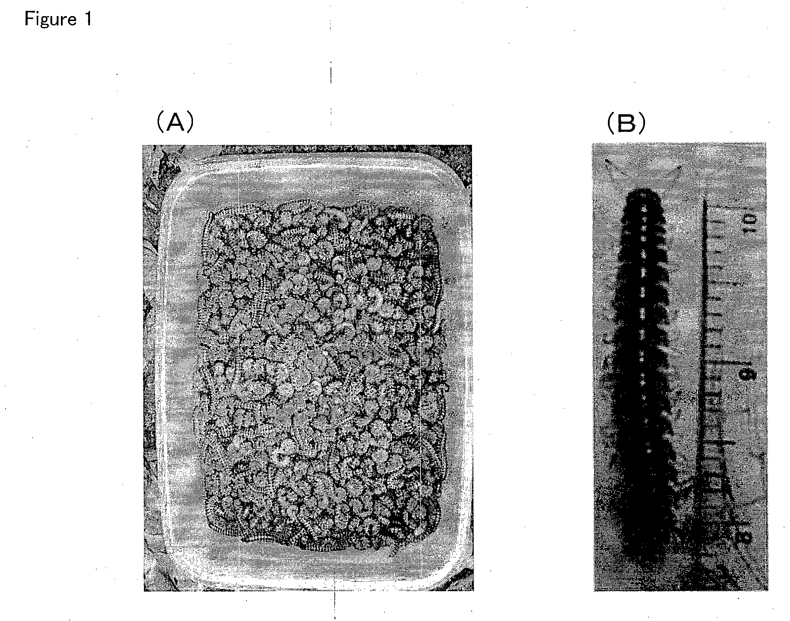

Chamberlinius hualienensis were caught in Kagoshima Prefecture of Japan during their plague in November 2010, November 2011 and August 2012. The caught Chamberlinius hualienensis were cooled by dry ice in a vessel and kept at - 80°C prior to use. Figure 1 are photographs of living Chamberlinius hualienensis.

The cryogenically preserved Chamberlinius hualienensis (total 2 kg) were grinded in liquid nitrogen by a mortar and a pestle. The obtained fine powder was suspended in 20 mM potassium phosphate buffer (pH 7.0) on ice by stirring for 3 hours or more. The resulting suspension was carefully passed through a few layers of cheesecloth to remove the solid substance. After the obtained solution was treated by protamine sulfate obtained from salmon sperm (manufactured by Nacalai Tesque, Inc.) for 30 minutes, the solution was centrifuged at 4°C and at 28500×g for 30 minutes. From the obtained supernatant, an enzyme was purified by the following condition. All of the procedures were performed at 0 to 4°C.

First, ammonium sulfate fractionation and subsequent column chromatography were employed to purify the (R)-hydroxynitrile lyase from the above-described supernatant. In the column chromatography, DEAE resin ("DEAE-TOYOPEARL (registered trademark) -650M" manufactured by Tosoh Corporation), hydrophobic resin ("Butyl-TOYOPEARL (registered trademark) -650M" manufactured by Tosoh Corporation), anion-exchange column ("Q-Sepharose FF" manufactured by GE Healthcare Bioscience), strong anion-exchange column ("MonoQTM 5/50 GL" manufactured by GE Healthcare Bioscience) and gel filtration chromatography column ("Superdex 75" and "Superdex 200 10/300GL" manufactured by GE Healthcare Bioscience) were used. An enzyme activity was measured in the following condition after every purification step. Hereinafter, the (R)-hydroxynitrile lyase purified from Chamberlinius hualienensis is abbreviated to "R-ChHNL".

### (2) Measurement of enzyme activity

The enzyme activity of the hydroxynitrile lyase was measured using benzaldehyde as a substrate as follows. Specifically, 1.25 M DMSO solution (40 µL) of benzaldehyde as a substrate and appropriate amount of enzyme solution (up to 100 µL) were mixed into 400 mM citrate buffer (pH 4.2, 760 µL). Then, 1 M KCN (100 µL) was added thereto to start the reaction, and the reaction was performed at 22°C for 5 minutes. After the reaction, 100 µL of the reaction mixture was taken out, and a mixed solution of n-hexane : 2-propanol = 85 : 15 was added thereto. The mixture was vigorously stirred and centrifuged at 4°C and at 16000×g for 3 minutes. The organic layer (500 µL) was recovered.

The obtained organic layer (5 µL) was analyzed by HPLC in the following condition.

Column: Chiral cell OJ-H column, manufactured by Daicel Corporation
Eluent: n-hexane : 2-propanol = 85 : 15
Flow rate: 1 mL/min
Column oven temperature: 30°C
Detection: 254 nm
Retention time: benzaldehyde - 5.5 min
   (R)-mandelonitrile - 12 min
   (S)-mandelonitrile - 14 min

The consumption of benzaldehyde as a substrate was monitored by absorbance determination at 280 nm. The enzyme data after every purification step are shown in Table 1.

**Table 1**

| Purification step | Enzyme activity (U) | Enzyme amount (mg) | Specific activity (U/mg) | Recovery rate (%) | Purification degree (fold) |
|---|---|---|---|---|---|
| Total homogenate | 26592 | 4387 | 6.06 | 100 | 1 |
| Ammonium sulfate fraction (50-70%) | 19608 | 1006 | 19.5 | 73.7 | 3.2 |
| DEAE | 11642 | 177 | 65.8 | 43.8 | 10.9 |
| Hydrophobic column | 5158 | 13.9 | 372.5 | 19.4 | 61.5 |
| Anion-exchange column | 1898 | 1.39 | 1369 | 7.14 | 226 |
| Strong anion-exchange column | 1268 | 0.46 | 2729 | 4.77 | 450 |
| Gel filtration | 892 | 0.12 | 7433 | 3.35 | 1226 |

In addition, the activity of decomposition reaction from racemic mandelonitrile to benzaldehyde was measured as follows. An enzyme solution was added into 100 mM citrate buffer (pH 5.0-5.5) containing 2 mM racemic mandelonitrile. The mixture was moderately stirred for the reaction at 22°C for 1 to 2 minutes, and the production amount of benzaldehyde was measured by absorbance at 280 nm. As the molecular extinction coefficient of benzaldehyde, ε₂₈₀ = 1.4 mM⁻¹cm⁻¹ was employed. The activity of the purified enzyme in cyanohydrin synthesis direction was 2.6 times higher than the activity in cyanohydrin decomposition reaction.

### Example 2: Structural analysis of natural (R)-hydroxynitrile lyase

### (1) Analysis of molecular mass and quaternary structure

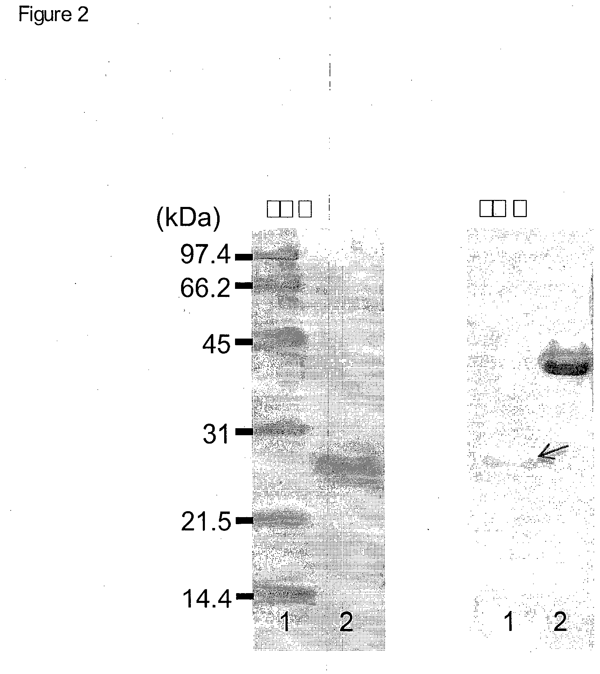

The size of monomer subunit of the R-ChHNL purified by the above-described Example 1 was analyzed by SDS-PAGE using molecular-weight marker (manufactured by Bio-Rad). The molecular mass and quaternary structure were obtained by gel filtration chromatography using "Superdex 10/300GL" manufactured by GE Healthcare. In detail, Dadashipour et al., Journal of Biotechnology, 153, pp.100-110 (2011) was referred to. Hereinafter, the document is abbreviated to "Dadashipour et al. (2011)". The result of SDS-PAGE is shown as Figure 2(A). In Figure 2(A), "1" is a lane of molecular markers of 97.4 kDa, 66.2 kDa, 45 kDa, 31 kDa, 21.5 kDa and 14.4 kDa from top down, and "2" is a lane of the purified R-ChHNL.

From the results of SDS-PAGE and gel filtration, it was confirmed that the molecular mass of the R-ChHNL is 47.3 kDa and the R-ChHNL is a homodimer of subunits having a molecular mass of 24.8 kDa.

### (2) Existence or non-existence of sugar chain

The existence or non-existence of sugar chain was confirmed using "Pierce Glycoprotein Staining kit" manufactured by Thermo Scientific K.K. Specifically, it was confirmed whether there was sugar chain or not by oxygenizing cis-diol contained in glycoprotein to an aldehyde with periodate and transforming the generated aldehyde group into Schiff base to develop magenta color in the SDS-PAGE gel. Figure 2(B) shows the result of the confirmation of whether R-ChHNL had sugar chain or not by the above-described reaction with the result of horseradish peroxidase, which is a typical glycoprotein. In Figure 2(B), "1" represents a lane of R-ChHNL and "2" represents a lane of horseradish peroxidase.

### (3) Spectroscopic analysis

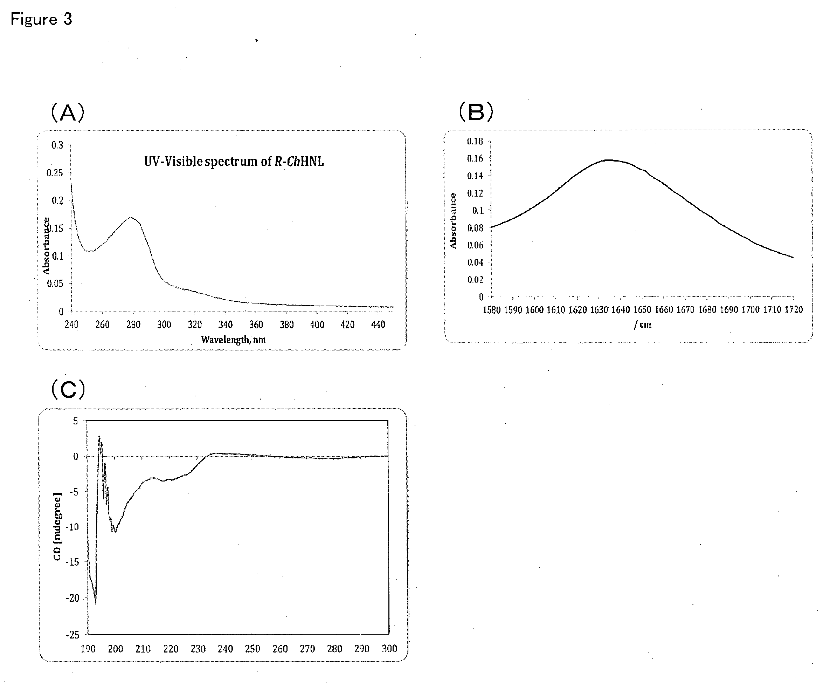

Into 20 mM potassium phosphate buffer (pH 7.0), R-ChHNL was dissolved. The solution was analyzed using a UV-Vis-NIR spectroscopy ("UV2600" manufactured by SHIMADZU Corporation) in the range of 210 to 600 nm to detect prosthetic group. In addition, R-ChHNL was analyzed using a spectrometer ("720 Circular Dichroism Spectrophotometer" manufactured by JASCO Corporation) in the range of 170 to 300 nm at room temperature and at pH 7.0. Furthermore, the secondary structure of the purified enzyme was estimated by using an infrared spectrophotometer ("Perkin-Elmer IR Spectrum 100" manufactured by Waltham). The ultraviolet·vislble·near-infrared spectrum, infrared spectrum and circular dichroism spectrum of R-ChHNL were measured. Each measurement result is shown as Figure 3(A) to (C).

On the basis of the measurement result of ultraviolet· visible·near-infrared spectrum (Figure 3(A)), R-ChHNL does not contain coenzyme FAD. On the basis of the measurement result of infrared spectrum (Figure 3(B)), it was found by the peak of 1645/cm that R-ChHNL is a β-rich protein. On the basis of the measurement result of circular dichroism spectrum (Figure 3(C)), it was found by circular dichroism of 222 nm that R-ChHNL is not α-rich.

### Example 3: Production of recombinant (R)-hydroxynitrile lyase

### (1) Cloning of cDNA encoding R-ChHNL

Chamberlinius hualienensis was anesthetized on ice, and segmental lateral extensions were collected using tweezers. The obtained tissues were added into a total RNA purification reagent ("TRIzol Reagent" manufactured by Invitrogen), and homogenaized with a disposable homogenizer ("BioMasher II" manufactured by Nippi, Incorporated). The RNA was extracted according to the manufacture's instruction. The cDNA for 5'/3'-RACE was synthesized using "SMART RACE cDNA Amplification Kit" and "SMARTScribe Reverse Transcriptase" manufactured by Clontech Laboratories, and subsequently treated with RNase H manufactured by Takara Bio Inc.

### (2) Design of primers

The amino acid sequence of the purified R-ChHNL was determined by Edman degradation with or without in-gel digestion method (APRO Life Science Institute). On the basis of the determined amino acid sequence, the following degenerate primers were designed.

PKAAINPIQEf: 5'-CC(A/C/G/T)AA(A/G)GC(A/C/G/T)GC(A/C/G/T)AT(A/T/C)AA(C/T)CC (A/C/G/T)AT(A/T/C)CA(A/G)GA-3' (SEQ ID NO:4)
APTALDIKf1: 5'-GC(A/C/G/T)CC(A/C/G/T)AC(A/C/G/T)GC(A/C/G/T)TT(A/G)GA(C/T)AT (A/C/T)AA-3' (SEQ ID NO:5)
APTALDIKf2: 5'-GC(A/C/G/T)CC(A/C/G/T)AC(A/C/G/T)GC(A/C/G/T)CT(A/C/G/T)GA(C/T)AT (A/C/T)AA-3' (SEQ ID NO:6)
APTALDIKr1: 5'-TT(A/G/T)AT(A/G)TC(C/T)AA(A/C/G/T)GC(A/C/G/T)GT(A/C/G/T)GG (A/C/G/T)GC-3' (SEQ ID NO:7)
APTALDIKr2: 5'-TT(A/G/T)AT(A/G)TC(A/C/G/T)AG(A/C/G/T)GC(A/C/G/T)GT(A/C/G/T)GG (A/C/G/T)GC-3' (SEQ ID NO:8)
AAINPIQEf: 5'-GC(A/C/G/T)GC(A/C/G/T)AT(A/C/T)AA(C/T)CC(A/C/G/T)AT(A/C/T)CA (A/G)GA-3' (SEQ ID NO:9)
ATINPIQEf: 5'-GC(A/C/G/T)AC(A/C/G/T)AT(A/C/T)AA(C/T)CC(A/C/G/T)AT(A/C/T)CA (A/G)GA-3' (SEQ ID NO:10)
LAINPIQEf1: 5'-TT(A/G)GC(A/C/G/T)AT(A/C/T)AA(C/T)CC(A/C/G/T)AT(A/C/T)CA (A/G)GA-3' (SEQ ID NO:11)
LAINPIQEf2: 5'-CT(A/C/G/T)GC(A/C/G/T)AT(A/C/T)AA(C/T)CC(A/C/G/T)AT(A/C/T)CA (A/G)GA-3' (SEQ ID NO:12)
LTINPIQEf1: 5'-TT(A/G)AC(A/C/G/T)AT(A/C/T)AA(C/T)CC(A/C/G/T)AT(A/C/T)CA (A/G)GA-3' (SEQ ID NO:13)
LTINPIQEf2: 5'-CT(A/C/G/T)AC(A/C/G/T)AT(A/C/T)AA(C/T)CC(A/C/G/T)AT(A/C/T)CA (A/G)GA-3' (SEQ ID NO:14)
AAINPIQEr: 5'-TC(C/T)TG(A/G/T)AT(A/C/G/T)GG(A/G)TT(A/G/T)AT(A/C/G/T)GC (A/C/G/T)GC-3' (SEQ ID NO:15)
ATINPIQEr: 5'-TC(C/T)TG(A/G/T)AT(A/C/G/T)GG(A/G)TT(A/G/T)AT(A/C/G/T)GT (A/C/G/T)GC-3' (SEQ ID NO:16)
LAINPIQEr1: 5'-TC(C/T)TG(A/G/T)AT(A/C/G/T)GG(A/G)TT(A/G/T)AT(A/C/G/T)GC (C/T)AA-3' (SEQ ID NO:17)
LAINPIQEr2: 5'-TC(C/T)TG(A/G/T)AT(A/C/G/T)GG(A/G)TT(A/G/T)AT(A/C/G/T)GC (A/C/G/T)AG-3' (SEQ ID NO:18)
LTINPIQEr1: 5'-TC(C/T)TG(A/G/T)AT(A/C/G/T)GG(A/G)TT(A/G/T)AT(A/C/G/T)GT (C/T)AA-3' (SEQ ID NO:19)
LTINPIQEr2: 5'-TC(C/T)TG(A/G/T)AT(A/C/G/T)GG(A/G)TT(A/G/T)AT(A/C/G/T)GT (A/C/G/T)AG-3' (SEQ ID NO:20)
NCPETHGCFAFf: 5'-AA(C/T)TG(C/T)CC(A/C/G/T)GA(A/G)AC(A/C/G/T)CA(C/T)GG(A/C/G/T) TG(C/T)TT(C/T)GC(A/C/G/T)TT-3' (SEQ ID NO:21)
NCPETHGCFAFr: 5'-AA(A/C/G/T)GC(A/G)AA(A/G)CA(A/C/G/T)CC(A/G)TG(A/C/G/T)GT (C/T)TC(A/C/G/T)GG(A/G)CA(A/G)TT-3' (SEQ ID NO:22)

The denotations before the sequences represent the amino acid sequences on which the degenerate primers are based, and the last "f" and "r" indicates the direction of the primer annealing for cDNA base sequence.

### (3) PCR

PCR was carried out by using the above-described degenerate primers, and polymerase ("Dream Taq DNA polymerase" manufactured by Thermo Fisher Scientific; and "Advantage GC2 Polymerase Mix" manufactured by Clontec Laboratories). The cycle of (i) at 94°C for 1 minute, (ii) at 40°C for 1 minute and (iii) at 72°C for 1 minute was repeated 70 times after at 94°C for 3 minutes for the reaction. Then, the PCR product was purified by using a gel ("Wizard SV PCR and Gel Clean-Up System" manufactured by Promega), and ligated into the Eco RV recognition site of a vector ("pBluescript II SK (+)" manufactured by Agilent Technologies). The DNA sequence was determined by using a gene analyzer ("3500 Genetic Analyzer" manufactured by Applied Biosystems). The obtained sequences were assembled and analyzed by using a sequence assembly software ("ATGC and Genetyx" manufactured by Genetyx).

On the basis of the obtained DNA sequences, the following gene-specific primers were designed.

R-ChHNL-1: 5'-CTGACTGAAA CCTTCGAATG CACCACTCG-3' (SEQ ID NO:23)
R-ChHNL-2: 5'-GGCATAATGA ATCTTGTCGC CGTTTGGAAC-3' (SEQ ID NO:24)
R-ChHNL-3: 5'-TTTGGTAGTG GACCAGCGAG CAGGTTGCAC-3' (SEQ ID NO:25)
R-ChHNL-4: 5'-ATAATCCCTT TAAAGTTCAG GTGCAATTAG-3' (SEQ ID NO:26)
R-ChHNL-5: 5'-ATACCAACAC ATCAAACTTA CCAAGCTTAG-3' (SEQ ID NO:27)
R-ChHNL-6: 5'-ATTATGGCTT ACGATTTCGT CGGTGGTCC-3' (SEQ ID NO:28)

PCR was carried out by using the above-described primers and DNA polymerase ("KOD plus neo" manufactured by TOYOBO CO., LTD.). As a template, the above-described cDNA prepared by using SMART RACE cDNA Amplification Kit was used. The cycle of (i) at 98°C for 10 seconds and (ii) at 68°C for 1 minute was repeated 35 times after at 94°C for 2 minutes for the reaction. The PCR product was ligated into a vector ("pBluescript II SK (+)" manufactured by Agilent Technologies) to determine the sequence. Other procedures were set as aforementioned. The full-length cDNA sequence was determined using 18 independent clones to avoid PCR-derived sequence errors.

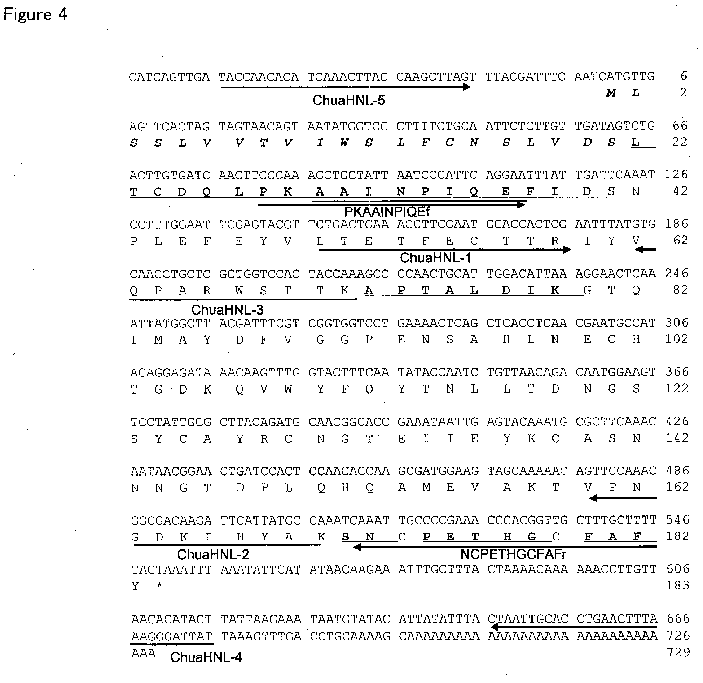

The base sequence of the obtained cDNA and the deduced amino acid sequence are shown in Figure 4. In Figure 4, the asterisk represents the stop codon, the signal peptide is described in italics, the arrow part represents the annealing site of the primer, and the underline of the amino acid residue represents the determined amino acid sequence. As described above, the cDNA which encodes the (R)-hydroxynitrile lyase (R-ChHNL) derived from Chamberlinius hualienensis was cloned. The deduced amino acid sequence does not share homology with any proteins found by Blastp search.

### (4) Construction of yeast expression vector

Using ChHNL-4 and ChHNL-5 as gene-specific primers and DNA polymerase ("PfuUltra II fusion HS DNA polymerase" manufactured by Agilent Technologies), the whole length of the cDNA was amplified. In the PCR, after the reaction at 95°C for 2 minutes, the cycle of (i) at 95°C for 20 seconds, (ii) at 40°C for 20 seconds and (iii) at 72°C for 1 minute was repeated 30 times and finally the reaction was performed at 72°C for 3 minutes. The reaction mixture was treated by restriction enzyme ("Dpn I" manufactured by New England Biolabs) at 37°C for 1 hour. The DNA fragment was purified by using a gel, and ligated into a vector ("pBluescript II SK (+)" by Stratagene) to determine the sequence as the above.

In order to insert the cDNA fragment which encodes mature ChHNL into a plasmid vector pPICZαA (manufactured by Life technologies), the following primers which contain restriction enzyme recognition site were designed.

XhoIkex2-mChuaHNL: 5'-GCGCTCGAGA AAAGACTGAC TTGTGATCAA CTTCCC-3' (SEQ ID NO:29)
ChuaHNLstop-XbaI: 5'-CGCTCTAGAT TAGTAAAAAG CAAAGCAACC GTGGGTTTC-3' (SEQ ID NO:30)

PCR was carried out using the above-described primers as the above, and the obtained PCR product was ligated into a vector ("pBluescript II SK (+)" manufactured by Stratagene) and the base sequence was determined. Then, the inserted sequence was ligated into the restriction enzyme recognition site of a plasmid vector ("pPICZαA" manufactured by Life Technologies).

### (5) Preparation of transformant and purification of recombinant R-ChHNL

The prepared plasmid vector was digested by using restriction enzyme SacI at 37°C for 3 to 4 hours. In order to obtain positive yeast clone, EasySelect Pichia Expression Kit (manufactured by Life technologies) and GS115 Pichia pastoris yeast were used. Pichia competent cell was prepared according to the method described in Joan Lin-Cereghino et al., BioTechniques, 38, pp.44-48 (2005). The transformant was cultivated in histidine added minimum glycerol medium, and then further cultivated in 4 L of histidine added minimum methanol (0.5%) medium 4L at 30°C for 4 days to induce the expression of the protein.

The culture medium was concentrated by using tangential flow filtration system, and added to anion exchange resin column (DEAE-TOYOPEARL (registered trademark) -650M, column volume: 25 mL). The non-adsorption fraction was subjected to hydrophobic interaction chromatography (Butyl-TOYOPEARL (registered trademark)-650M), and further R-ChHNL was purified by Superdex 10/300GL.

### Example 4: Substrate of R-ChHNL

The substrate of R-ChHNL was identified by cyanohydrin synthetic reaction. In order to identify the substrate of R-ChHNL, 2.5 U of strong anion exchange column fraction (25 µL, Table 1) was used as an enzyme sample and redistilled water was used instead of the enzyme sample as blank. The enzyme sample, 300 µmol of sodium citrate buffer (pH 4.2), 50 mM carbonyl compound and 100 mM KCN were mixed (total volume: 1 mL) to be reacted at 25°C for 5 minutes. It was determined whether the enzyme had an activity or not by measuring wavelength of 254 nm with HPLC using OJ-H chiral column to detect the reaction product.

In regard to tested aromatic compounds other than benzaldehyde, 50 mM DMSO solutions (4 µL) were prepared for the reaction. A mixed solvent of n-hexane : 2-propanol = 19 : 1 was used as an extraction solvent for tested 4-bromobenzaldehyde. When a tested compound was not dissolved in water, the mixture was stirred at 30°C and at 1000 to 1500 rpm. In case of an aliphatic tested compound, a reaction time was adjusted to 2 hours. After the reaction, 600 µL of diisopropyl ether was added to 400 µL of the reaction mixture. The mixture was vigorously stirred and centrifuged at 16000 g for 5 minutes. Into 400 µL of the supernatant, 20 µL of acetic anhydride, 10 µL of pyridine and 2 to 3 mg of 4-dimethylaminopyridine were added for the reaction at 37°C overnight. Subsequently, the reaction at 60°C for 2 to 4 hours was performed to vaporize a cyanohydrin compound and 4-dimethylaminopyridine. Further, 100 µL of water and 400 µL of ethyl acetate were added thereto. The mixture was centrifuged, and 150 µL of the supernatant was used as a sample. For the above procedures, references were made to Effenberger, Stelzer, Tetrahedron: Asymmetry, 6, pp.283-286 (1995). In order to detect the generated compound, gas chromatography ("GC-2014" manufactured by SHIMADZU Corporation) to which Supelco β-Dex 325 column was attached was used, and helium was used as carrier gas. The compounds on which the activity was confirmed are shown in Table 2.

**Table 2**

| Aromatic carbonyl compound | Aliphatic carbonyl compound |
|---|---|
| benzaldehyde | trans-2-methyl-2-butenal |
| 2-chlorobenzaldehyde | 2-heptanone |
| 4-chlorobenzaldehyde | 2-hexanone |
| 4-bromobenzaldehyde | crotonaldehyde |
| 4-fluorobenzaldehyde | trans-2-hexenal |
| 2-methylbenzaldehyde | n-octanal |
| 3-methylbenzaldehyde | trans-2-methyl-2-pentenal |
| 4-methylbenzaldehyde | n-heptanal |
| 3-methoxybenzaldehyde | 3-methyl-2-butenal |
| 4-methoxybenzaldehyde | propanal |
| 3-nitrobenzaldehyde | isobutylaldehyde |
| 4-nitrobenzaldehyde | pivalaldehyde |
| terephthalaldehyde | 2-butanone |
| 4-biphenyldicarboxaldehyde | 3-methyl-2-butanone |
| 2,3-dichlorobenzaldehyde | 2-hexanone |
| 2,4-dichlorobenzaldehyde | 2-methyl-3-pentanone |
| 2,5-dichlorobenzaldehyde | |
| 3,4-dichlorobenzaldehyde | |
| piperonal | |
| 2-furancarbaldehyde | |
| 2-thiophenecarbaldehyde | |
| 1-naphthalenecarbaldehyde | |
| 2-naphthalenecarbaldehyde | |

As the result shown in Table 2, it was demonstrated that R-ChHNL recognizes not only benzaldehyde, which is recognized as a substrate by a general natural HNL, but also various carbonyl compounds as a substrate.

### Example 5: Dynamic analysis of R-ChHNL

Various aldehyde compounds or racemic mandelonitrile was used as a substrate, and an enzyme reaction was performed in a condition similar to the above-described condition. However, when benzaldehyde was used as a substrate, with respect to the reaction condition, the pH was adjusted to 5.2 and the temperature was adjusted to 22°C or the pH was adjusted to 5.8 and the temperature was adjusted to 35°C. A substrate saturation curve was prepared by using benzaldehyde in various concentrations as a substrate, performing each enzyme activity measurement 3 times and calculating the average from the measurements, and Hanes-Woolf plot was used to evaluate the values of Vₘₐₓ, Kₘ and k_{cat}. The results are shown in Table 3. In Table 3, "S.A." represents specific activity. The relative specific activity means a specific activity relative to the case that benzaldehyde was used as a substrate to perform the reaction at pH 5.2 and 22°C. When mandelonitrile was used as a substrate, decomposition reaction was caused; therefore, specific activity could not be calculated.

**Table 3**

| Substrate | Kₘ (mM) | Vₘₐₓ (µmol/min·mg) | k_{cat} (/s) | k_{cat}/Kₘ (/mM·s) | max S.A. (U/mg) | relative S.A. |
|---|---|---|---|---|---|---|
| benzaldehyde (pH5.2, 22°C) | 3.2 | 8185 | 3390 | 1064 | 7433 | 100 |
| benzaldehyde (pH5.8, 35°C) | 17 | 20530 | 8503 | 501 | 17141 | 230.6 |
| piperonal | 5.6 | 3006.6 | 1245.2 | 222.7 | 2748 | 37.0 |
| 2,4-dimethylbenzaldehyde | 0.94 | 632.2 | 261.8 | 278 | 654 | 8.8 |
| 3-methoxybenzaldehyde | 5.7 | 3228.2 | 1337 | 233.3 | 3326 | 44.7 |
| 2-methylbenzaldehyde | 3.1 | 2564.9 | 1062.3 | 347 | 2395 | 32.2 |
| 3-methylbenzaldehyde | 9.9 | 14002.1 | 5799.2 | 583.8 | 12657 | 170.3 |
| 4-methylbenzaldehyde | 5.0 | 11918.4 | 4936.2 | 989 | 11072 | 149.0 |
| 2-thiophenecarboxaldehyde | 18.7 | 3522 | 1459 | 78 | 2403 | 32.3 |
| 4-methoxybenzaldehyde | 6.7 | 7482 | 3099 | 466 | 6849 | 92.1 |
| 1-naphthalenecarboxaldehyde | 0.65 | 313 | 130 | 199 | 326 | 4.4 |
| 4-bromobenzaldehyde | 9.5 | 19940 | 8259 | 871 | 17375 | 233.8 |
| racemic mandelonitrile | 5.5 | 2819 | 1168 | 211 | 1896 | - |

As the result shown in Table 3, R-ChHNL exhibits the highest k_{cat}/Kₘ value in comparison with conventionally-known HNL, though R-ChHNL does not utilize FAD as a coenzyme.

### Example 6: Stability of R-ChHNL to temperature and pH

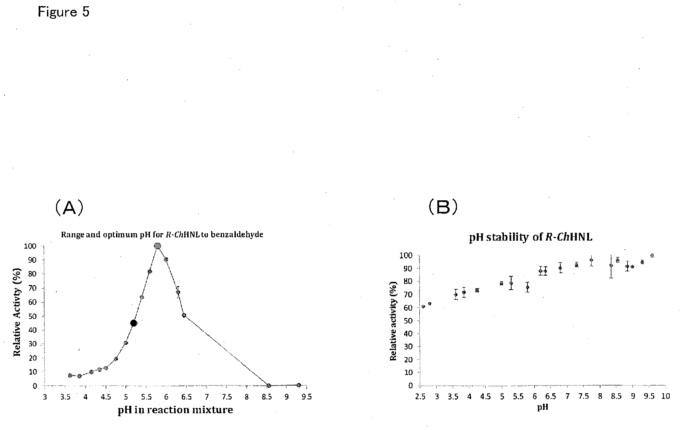

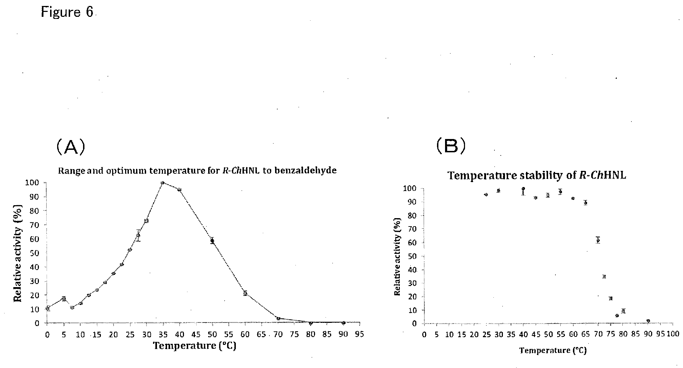

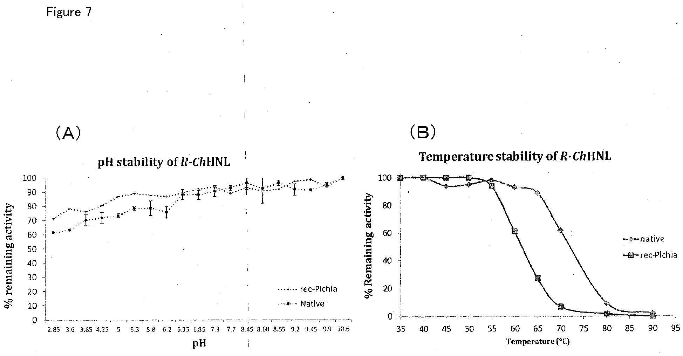

With respect to the natural R-ChHNL obtained in the above-described Example 1 and recombinant R-ChHNL obtained in the above-described Example 3, the stability to temperature and pH was tested by using a citrate buffer of which final concentration was 400 mM and benzaldehyde as a substrate and adjusting the enzyme reaction condition to be similar to the above. The stability to temperature was measured by performing the reaction in a reaction mixture of pH 7.0 at the temperature range of 0 to 70°C for 1 hour. In addition, the reaction was similarly performed except that the reaction temperature was adjusted to 20°C and pH was changed in the range of 3 to 11. In the reaction, citrate-phosphate buffer was used when pH was 3 to 8, and glycine-NaOH buffer was used when pH was 8 to 11. The enzyme activity was measured according to the method described in Dadashipour et al. (2011). The relations between the reaction pH and the specific activity and between the reaction pH and the remaining activity of the natural R-ChHNL are respectively shown in Figure 5(A) and Figure 5(B), and the relations between the reaction temperature and the specific activity and between the reaction temperature and the remaining activity of the natural R-ChHNL are respectively shown in Figure 6(A) and Figure 6(B). In addition, the relation between the reaction temperature and the remaining activity and the relation between the reaction pH and the remaining activity of both R-ChHNLs are respectively shown in Figure 7(A) and Figure 7(B). In Figure 7, "rec-Pichia" represents recombinant R-ChHNL.

As Figure 5, the natural R-ChHNL exhibits maximum activity at pH 5.8 and about 10% of maximum activity value even at pH 4. The optimum pH value is higher than 5.1 as optimum pH value of HNL derived from other plant. In addition, as Figure 5(B), the natural R-ChHNL has stability in broad pH range.

Furthermore, as Figure 6, it was demonstrated that the natural R-ChHNL exhibits activity in broad temperature range of 0 to 70°C, has optimum temperature of 35°, and exhibits high activity at 60°C or lower. It is described in Woker, R. et al., Methods Enzymol., 228, pp.584-590 (1994); and Jansen, I. et al., Biotechnol. Appl. Biochem., 15, pp.90-99 (1992) that the HNL derived from almond has the highest stability in conventionally-known HNLs derived from a plant and is stable when the HNL is maintained at 60°C for 1 hour. However, even such a most highly-stable HNL derived from almond is completely inactivated at 75°C for 30 minutes. On the one hand, the activity of the R-ChHNL according to the present invention may be maintained even at 60°C for 1 hour or longer as Figure 6, and 20% of the activity is maintained even at 75°C for 1 hour. Thus, it is clear that the R-ChHNL of the present invention has higher stability than conventionally-known HNLs.

In addition, the recombinant R-ChHNL is a little less stable to temperature than the natural R-ChHNL as Figure 7, but the stability to pH of the recombinant R-ChHNL is equal to or superior to the natural R-ChHNL and has sufficiently high stability in comparison with a general enzyme.

### Example 7: Search of inhibitor of R-ChHNL activity

With respect to an inhibitor of R-ChHNL activity, an experiment was conducted. The reaction was performed in the condition similar to the above-described enzyme reaction condition. Specifically, the natural R-ChHNL obtained in Example 1 was used, and test compound was added to the reaction mixture in the concentration of 1 mM or 0.1 mM in 10 mM potassium phosphate buffer (pH 7.0) at 20°C for 60 minutes. The remaining activity was measured 3 times. The average values are shown in Table 4.

**Table 4**

| Inhibitor candidate | Remaining activity | Inhibitor candidate | Remaining activity |
|---|---|---|---|
| **Sulfhydryl compound** | | **Biotin reagent** | |
| N-ethylmaleimide | 100% | avidin (100 µg/mL) | 96% |
| dithionitrobenzoic acid | 97% | **Chelating agent** | |
| iodoacetic acid (1mM or 10mM) | 52%, 18% | EDTA (10mM) | 100% |
| iodoacetamide (1mM or 10mM) | 75%, 28% | barbital sodium | 93% |
| *p*-chloromercuribenzoic acid | 100% | **Metal salt** | |

| **His modifier** | | PbCl₂ (0.1 mM or 1mM) | 98%, 77% |
|---|---|---|---|
| diethylpyrocarbonate (1 mM or 10 mM) | 100%, 79% | NiCl₂ | 93% |

| **Serine protease inhibitor** | | CoCl₂ | 95% |
|---|---|---|---|
| trypsin inhibitor (T-9003) | 88% | CrCl₃ | 88% |
| trypsin inhibitor (T-9378) | 85% | CuSO₄ | 100% |
| trypsin-chymotrypsin inhibitor (T-9777) | 91% | FeCl₃ | 100% |
| phenylmethylsulfonyl fluoride (PMSF) (1mM or 10mM) | 91%, 60% | HgCl₂ | 64% |

| **Aspartic protease inhibitor** | | ZnSO₄ | 100% |
|---|---|---|---|
| pepstatin A (100 µg/mL) | 89% | CdCl₂ | 99% |
| Serine, cysteine, threonine peptidase inhibitor | | MnSO₄ | 106% |
| leupeptin (100 µg/mL) | 86% | AgNO₃ | 68% |

| **Reducing agent** | | Others | |
|---|---|---|---|
| 2-mercaptoethanol (10 mM) | 95% | ammonium thiocyanate (1mM) | 43% |
| hydrazine | 100% | phenylthiourea | 100% |
| DTT | 100% | diphenylhydantoin | 100% |
| oxidized glutathione (0.5 mM) | 95% | | |
| reduced glutathione (0.5 mM) | 93% | | |

As the result shown in Table 4, only a few compound exhibit inhibitory activity among test compounds. For example, iodoacetic acid and iodoacetamide which are sulfhydryl reagents inhibit the activity of R-ChHNL, when the concentration thereof is increased to 10 mM. It was confirmed that PMSF which is a famous serine protease inhibitor denotes the same tendency. Ammonium thiocyanate strongly inhibits S-HNL derived from Para rubber tree (Hevea brasiliensis) and Baliospermum, which are plants belonging to the euphorbia family, but does not inhibit the R-ChHNL according to the present invention. Among metals, only mercury ion and silver ion exhibit slight inhibitory activity as 30 to 40%. As the above results, it was demonstrated that the R-ChHNL according to the present invention exhibits stable activity even in the presence of various inhibitors of conventional enzymes' activity.

## Claims

1. A (R)-hydroxynitrile lyase having any one of the following amino acid sequences (1) to (3):
(1) an amino acid sequence of SEQ ID NO: 3;
(2) an amino acid sequence specified in the (1) with deletion, substitution and/or addition of 1 or more and 30 or less amino acid residues, wherein a hydroxynitrile lyase activity of a (R)-hydroxynitrile lyase having the amino acid sequence (2) is the same as or superior to that of natural (R)-hydroxynitrile lyase having the amino acid sequence of SEQ ID NO: 3;
(3) an amino acid sequence having at least 70% sequence homology to the amino acid sequence specified in the (1), wherein a hydroxynitrile lyase activity of a (R)-hydroxynitrile lyase having the amino acid sequence (3) is the same as or superior to that of natural (R)-hydroxynitrile lyase having the amino acid sequence of SEQ ID NO: 3.

2. The (R)-hydroxynitrile lyase according to claim 1, being a dimer of a subunit having any one of the amino acid sequences (1) to (3).

3. The (R)-hydroxynitrile lyase according to claim 1 or 2, derived from genus *Chamberlinius.*

4. The (R)-hydroxynitrile lyase according to claim 3, derived from *Chamberlinius hualienensis.*

5. A (R)-hydroxynitrile lyase gene having any one of the following base sequences (4) to (6):
(4) a base sequence of SEQ ID NO: 2;
(5) a base sequence encoding the amino acid sequence (2) according to claim 1;
(6) a base sequence encoding the amino acid sequence (3) according to claim 1.

6. The (R)-hydroxynitrile lyase gene according to claim 5, having a base sequence of SEQ ID NO: 1.

7. A vector, comprising the (R)-hydroxynitrile lyase gene according to claim 5 or 6.

8. A transformant, transformed by the vector according to claim 7.

9. A method for producing a (R)-hydroxynitrile lyase, comprising the steps of:
cultivating the transformant according to claim 8 to obtain a culture, and
purifying the (R)-hydroxynitrile lyase from the culture.

## Patentansprüche

1. (R)-Hydroxynitrillyase, die eine der folgenden Aminosäuresequenzen (1) bis (3) aufweist:
(1) eine Aminosäuresequenz nach SEQ ID NO: 3,
(2) eine Aminosäuresequenz wie in (1) spezifiziert mit Deletion, Substitution und/oder Addition von 1 oder mehr und 30 oder weniger Aminosäureresten, wobei eine Hydroxynitrillyaseaktivität einer (R)-Hydroxynitrillyase, welche die Aminosäuresequenz (2) aufweist, gleich oder besser ist, als die einer natürlichen (R)-Hydroxynitrillyase, welche die Aminosäuresequenz nach SEQ ID NO: 3 aufweist,
(3) eine Aminosäuresequenz, die mindestens 70% Sequenzhomologie zu der in (1) spezifizierten Aminosäuresequenz aufweist, wobei eine Hydroxynitrillyaseaktivität einer (R)-Hydroxynitrillyase, welche die Aminosäuresequenz (3) aufweist, gleich oder besser ist, als die einer natürlichen (R)-Hydroxynitrillyase, welche die Aminosäuresequenz nach SEQ ID NO: 3 aufweist.

2. (R)-Hydroxynitrillyase nach Anspruch 1, welche ein Dimer einer Untereinheit ist, die eine der Aminosäuresequenzen (1) bis (3) aufweist.

3. (R)-Hydroxynitrillyase nach Anspruch 1 oder 2, die von der Gattung *Chamberlinius* stammt.

4. (R)-Hydroxynitrillyase nach Anspruch 3, die von *Chamberlinius hualienensis* stammt.

5. (R)-Hydroxynitrillyasegen, das eine der folgenden Basensequenzen (4) bis (6) aufweist:
(4) eine Basensequenz nach SEQ ID NO: 2,
(5) eine Basensequenz, welche die Aminosäuresequenz (2) nach Anspruch 1 kodiert,
(6) eine Basensequenz, welche die Aminosäuresequenz (3) nach Anspruch 1 kodiert.

6. (R)-Hydroxynitrillyasegen nach Anspruch 5, das eine Basensequenz nach SEQ ID NO: 1 aufweist.

7. Vektor, umfassend das (R)-Hydroxynitrillyasegen nach Anspruch 5 oder 6.

8. Transformante, die durch den Vektor nach Anspruch 7 transformiert ist.

9. Verfahren zur Herstellung einer (R)-Hydroxynitrillyase, umfassend die Schritte:
Kultivieren der Transformante nach Anspruch 8, um eine Kultur zu erhalten und Aufreinigen der (R)-Hydroxynitrillyase aus der Kultur.

## Revendications

1. (R)-Hydroxynitrile lyase ayant l'une quelconque des séquences d'acides aminés (1) à (3) suivantes :
(1) une séquence d'acides aminés de SEQ ID NO : 3 ;
(2) une séquence d'acides aminés spécifiée en (1) avec la délétion, la substitution et/ou l'addition de 1 ou plus et de 30 ou moins de résidus d'acides aminés, dans laquelle une activité hydroxynitrile lyase d'une (R)-hydroxynitrile lyase ayant la séquence d'acides aminés (2) est identique ou supérieure à celle de la (R)-hydroxynitrile lyase naturelle ayant la séquence d'acides aminés de SEQ ID NO : 3 ;
(3) une séquence d'acides aminés présentant au moins 70 % d'homologie de séquence avec la séquence d'acides aminés spécifiée en (1), dans laquelle une activité hydroxynitrile lyase d'une (R)-hydroxynitrile lyase ayant la séquence d'acides aminés (3) est identique ou supérieure à celle de la (R)-hydroxynitrile lyase naturelle ayant la séquence d'acides aminés de SEQ ID NO : 3.

2. (R)-Hydroxynitrile lyase selon la revendication 1, étant un dimère d'une sous-unité ayant l'une quelconque des séquences d'acides aminés (1) à (3) .

3. (R)-Hydroxynitrile lyase selon la revendication 1 ou 2, dérivée du genre *Chamberlinius.*

4. (R)-Hydroxynitrile lyase selon la revendication 3, dérivée de *Chamberlinius hualienensis.*

5. Gène de la (R)-hydroxynitrile lyase ayant l'une quelconque des séquences de bases (4) à (6) suivantes :
(4) une séquence de bases de SEQ ID NO : 2 ;
(5) une séquence de bases codant pour la séquence d'acides aminés (2) selon la revendication 1 ;
(6) une séquence de bases codant pour la séquence d'acides aminés (3) selon la revendication 1.

6. Gène de la (R)-hydroxynitrile lyase selon la revendication 5, ayant une séquence de bases de SEQ ID NO : 1.

7. Vecteur, comprenant le gène de la (R)-hydroxynitrile lyase selon la revendication 5 ou 6.

8. Transformant, transformé par le vecteur selon la revendication 7.

9. Procédé de production d'une (R)-hydroxynitrile lyase, comprenant les étapes de :
la culture du transformant selon la revendication 8 pour obtenir une culture, et
la purification de la (R)-hydroxynitrile lyase à partir de la culture.


### SEQUENCE LISTING

<110> TOYAMA PREFECTURE
<120> (R)-HYDROXYNITRILELYASE
<130> F15-013PCT
<150> JP 2014-42181
   <151> 2014-3-4
<160> 30
<210> 1
   <211> 783
   <212> DNA
   <213> Chamberlinius hualienensis
<400> 1
   CATCAGTTGA TACCAACACA TCAAACTTAC CAAGCTTAGT TTACGATTTC AATCATGTTG    60
   AGTTCACTAG TAGTAACAGT AATATGGTCG CTTTTCTGCA ATTCTCTTGT TGATAGTCTG   120
   ACTTGTGATC AACTTCCCAA AGCTGCTATT AATCCCATTC AGGAATTTAT TGATTCAAAT   180
   CCTTTGGAAT TCGAGTACGT TCTGACTGAA ACCTTCGAAT GCACCACTCG AATTTATGTG   240
   CAACCTGCTC GCTGGTCCAC TACCAAAGCC CCAACTGCAT TGGACATTAA AGGAACTCAA   300
   ATTATGGCTT ACGATTTCGT CGGTGGTCCT GAAAACTCAG CTCACCTCAA CGAATGCCAT   360
   ACAGGAGATA AACAAGTTTG GTACTTTCAA TATACCAATC TGTTAACAGA CAATGGAAGT   420
   TCCTATTGCG CTTACAGATG CAACGGCACC GAAATAATTG AGTACAAATG CGCTTCAAAC   480
   AATAACGGAA CTGATCCACT CCAACACCAA GCGATGGAAG TAGCAAAAAC AGTTCCAAAC   540
   GGCGACAAGA TTCATTATGC CAAATCAAAT TGCCCCGAAA CCCACGGTTG CTTTGCTTTT   600
   TACTAAATTT AAATATTCAT ATAACAAGAA ATTTGCTTTA CTAAAACAAA AAACCTTGTT   660
   AACACATACT TATTAAGAAA TAATGTATAC ATTATATTTA CTAATTGCAC CTGAACTTTA   720
   AAGGGATTAT TAAAGTTTGA CCTGCAAAAG CAAAAAAAAA AAAAAAAAAA AAAAAAAAAA   780
   AAA                                                                 783
<210> 2
   <211> 552
   <212> DNA
   <213> Chamberlinius hualienensis
<400> 2
   ATGTTGAGTT CACTAGTAGT AACAGTAATA TGGTCGCTTT TCTGCAATTC TCTTGTTGAT     60
   AGTCTGACTT GTGATCAACT TCCCAAAGCT GCTATTAATC CCATTCAGGA ATTTATTGAT    120
   TCAAATCCTT TGGAATTCGA GTACGTTCTG ACTGAAACCT TCGAATGCAC CACTCGAATT    180
   TATGTGCAAC CTGCTCGCTG GTCCACTACC AAAGCCCCAA CTGCATTGGA CATTAAAGGA    240
   ACTCAAATTA TGGCTTACGA TTTCGTCGGT GGTCCTGAAA ACTCAGCTCA CCTCAACGAA    300
   TGCCATACAG GAGATAAACA AGTTTGGTAC TTTCAATATA CCAATCTGTT AACAGACAAT    360
   GGAAGTTCCT ATTGCGCTTA CAGATGCAAC GGCACCGAAA TAATTGAGTA CAAATGCGCT    420
   TCAAACAATA ACGGAACTGA TCCACTCCAA CACCAAGCGA TGGAAGTAGC AAAAACAGTT    480
   CCAAACGGCG ACAAGATTCA TTATGCCAAA TCAAATTGCC CCGAAACCCA CGGTTGCTTT    540
   GCTTTTTACT AA                                                        552
<210> 3
   <211> 162
   <212> PRT
   <213> Chamberlinius hualienensis
<400> 3
   Leu Thr Cys Asp Gln Leu Pro Lys Ala Ala Ile Asn Pro Ile Gln
                   5                   10                  15
   Glu Phe Ile Asp Ser Asn Pro Leu Glu Phe Glu Tyr Val Leu Thr
                   20                  25                  30
   Glu Thr Phe Glu Cys Thr Thr Arg Ile Tyr Val Gln Pro Ala Arg
                   35                  40                  45
   Trp Ser Thr Thr Lys Ala Pro Thr Ala Leu Asp Ile Lys Gly Thr
                   50                  55                  60
   Gln Ile Met Ala Tyr Asp Phe Val Gly Gly Pro Glu Asn Ser Ala
                   65                  70                  75
   His Leu Asn Glu Cys His Thr Gly Asp Lys Gln Val Trp Tyr Phe
                   80                  85                  90
   Gln Tyr Thr Asn Leu Leu Thr Asp Asn Gly Ser Ser Tyr Cys Ala
                   95                  100                 105
   Tyr Arg Cys Asn Gly Thr Glu Ile Ile Glu Tyr Lys Cys Ala Ser
                   110                 115                 120
   Asn Asn Asn Gly Thr Asp Pro Leu Gln His Gln Ala Met Glu Val
                   125                 130                 135
   Ala Lys Thr Val Pro Asn Gly Asp Lys Ile His Tyr Ala Lys Ser
                   140                 145                 150
   Asn Cys Pro Glu Thr His Gly Cys Phe Ala Phe Tyr
                   155                 160
<210> 4
   <211> 29
   <212> DNA
   <213> Artificial
<220>
   <221> CDS
   <222> 1..29
   <223> oligonucleotide primer
<400> 4
   CCNAARGCNG CNATHAAYCC NATHCARGA 29
<210> 5
   <211> 23
   <212> DNA
   <213> Artificial
<220>
   <221> CDS
   <222> 1..23
   <223> oligonucleotide primer
<400> 5
   GCNCCNACNG CNTTRGAYAT HAA 23
<210> 6
   <211> 23
   <212> DNA
   <213> Artificial
<220>
   <221> CDS
   <222> 1..23
   <223> oligonucleotide primer
<400> 6
   GCNCCNACNG CNCTNGAYAT HAA 23
<210> 7
   <211> 23
   <212> DNA
   <213> Artificial
<220>
   <221> CDS
   <222> 1..23
   <223> oligonucleotide primer
<400> 7
   TTDATRTCYA ANGCNGTNGG NGC 23
<210> 8
   <211> 23
   <212> DNA
   <213> Artificial
<220>
   <221> CDS
   <222> 1..23
   <223> oligonucleotide primer
<400> 8
   TTDATRTCNA GNGCNGTNGG NGC 23
<210> 9
   <211> 23
   <212> DNA
   <213> Artificial
<220>
   <221> CDS
   <222> 1..23
   <223> oligonucleotide primer
<400> 9
   GCNGCNATHA AYCCNATHCA RGA 23
<210> 10
   <211> 23
   <212> DNA
   <213> Artificial
<220>
   <221> CDS
   <222> 1..23
   <223> oligonucleotide primer
<400> 10
   GCNACNATHA AYCCNATHCA RGA 23
<210> 11
   <211> 23
   <212> DNA
   <213> Artificial
<220>
   <221> CDS
   <222> 1..23
   <223> oligonucleotide primer
<400> 11
   TTRGCNATHA AYCCNATHCA RGA 23
<210> 12
   <211> 23
   <212> DNA
   <213> Artificial
<220>
   <221> CDS
   <222> 1..23
   <223> oligonucleotide primer
<400> 12
   CTNGCNATHA AYCCNATHCA RGA 23
<210> 13
   <211> 23
   <212> DNA
   <213> Artificial
<220>
   <221> CDS
   <222> 1..23
   <223> oligonucleotide primer
<400> 13
   TTRACNATHA AYCCNATHCA RGA 23
<210> 14
   <211> 23
   <212> DNA
   <213> Artificial
<220>
   <221> CDS
   <222> 1..23
   <223> oligonucleotide primer
<400> 14
   CTNACNATHA AYCCNATHCA RGA 23
<210> 15
   <211> 23
   <212> DNA
   <213> Artificial
<220>
   <221> CDS
   <222> 1..23
   <223> oligonucleotide primer
<400> 15
   TCYTGDATNG GRTTDATNGC NGC 23
<210> 16
   <211> 23
   <212> DNA
   <213> Artificial
<220>
   <221> CDS
   <222> 1..23
   <223> oligonucleotide primer
<400> 16
   TCYTGDATNG GRTTDATNGT NGC 23
<210> 17
   <211> 23
   <212> DNA
   <213> Artificial
<220>
   <221> CDS
   <222> 1..23
   <223> oligonucleotide primer
<400> 17
   TCYTGDATNG GRTTDATNGC YAA 23
<210> 18
   <211> 23
   <212> DNA
   <213> Artificial
<220>
   <221> CDS
   <222> 1..23
   <223> oligonucleotide primer
<400> 18
   TCYTGDATNG GRTTDATNGC NAG 23
<210> 19
   <211> 23
   <212> DNA
   <213> Artificial
<220>
   <221> CDS
   <222> 1..23
   <223> oligonucleotide primer
<400> 19
   TCYTGDATNG GRTTDATNGT YAA 23
<210> 20
   <211> 23
   <212> DNA
   <213> Artificial
<220>
   <221> CDS
   <222> 1..23
   <223> oligonucleotide primer
<400> 20
   TCYTGDATNG GRTTDATNGT NAG 23
<210> 21
   <211> 32
   <212> DNA
   <213> Artificial
<220>
   <221> CDS
   <222> 1..32
   <223> oligonucleotide primer
<400> 21
   AYTGYCCNG ARACNCAYGG NTGYTTYGCN TT 32
<210> 22
   <211> 32
   <212> DNA
   <213> Artificial
<220>
   <221> CDS
   <222> 1..32
   <223> oligonucleotide primer
<400> 22
   AANGCRAARC ANCCRTGNGT YTCNGGRCAR TT 32
<210> 23
   <211> 29
   <212> DNA
   <213> Artificial
<220>
   <221> CDS
   <222> 1..29
   <223> oligonucleotide primer
<400> 23
   CTGACTGAAA CCTTCGAATG CACCACTCG 29
<210> 24
   <211> 30
   <212> DNA
   <213> Artificial
<220>
   <221> CDS
   <222> 1..30
   <223> oligonucleotide primer
<400> 24
   GGCATAATGA ATCTTGTCGC CGTTTGGAAC 30
<210> 25
   <211> 30
   <212> DNA
   <213> Artificial
<220>
   <221> CDS
   <222> 1..30
   <223> oligonucleotide primer
<400> 25
   TTTGGTAGTG GACCAGCGAG CAGGTTGCAC 30
<210> 26
   <211> 30
   <212> DNA
   <213> Artificial
<220>
   <221> CDS
   <222> 1..30
   <223> oligonucleotide primer
<400> 26
   ATAATCCCTT TAAAGTTCAG GTGCAATTAG 30
<210> 27
   <211> 30
   <212> DNA
   <213> Artificial
<220>
   <221> CDS
   <222> 1..30
   <223> oligonucleotide primer
<400> 27
   ATACCAACAC ATCAAACTTA CCAAGCTTAG 30
<210> 28
   <211> 29
   <212> DNA
   <213> Artificial
<220>
   <221> CDS
   <222> 1..29
   <223> oligonucleotide primer
<400> 28
   ATTATGGCTT ACGATTTCGT CGGTGGTCC 29
<210> 29
   <211> 36
   <212> DNA
   <213> Artificial
<220>
   <221> CDS
   <222> 1..36
   <223> oligonucleotide primer
<400> 29
   GCGCTCGAGA AAAGACTGAC TTGTGATCAA CTTCCC 36
<210> 30
   <211> 39
   <212> DNA
   <213> Artificial
<220>
   <221> CDS
   <222> 1..39
   <223> oligonucleotide primer
<400> 30
   CGCTCTAGAT TAGTAAAAAG CAAAGCAACC GTGGGTTTC 39